(19) Europäisches Patentamt
European Patent Office
Office européen des brevets

(11) **EP 2 522 987 A1**

(12) **EUROPEAN PATENT APPLICATION**
published in accordance with Art. 153(4) EPC

(43) Date of publication:
**14.11.2012 Bulletin 2012/46**

(51) Int Cl.:
**G01N 21/64** (2006.01)  **G01N 15/14** (2006.01)
**G01N 33/483** (2006.01)

(21) Application number: **11731752.9**

(22) Date of filing: **05.01.2011**

(86) International application number:
**PCT/JP2011/000004**

(87) International publication number:
**WO 2011/083754 (14.07.2011 Gazette 2011/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2010 JP 2010001257**

(71) Applicant: **Mitsui Engineering & Shipbuilding Co., Ltd.**
**Chuo-ku**
**Tokyo 104-8439 (JP)**

(72) Inventors:
• **HAYASHI, Hironori**
**Tamano-shi**
**Okayama 706-8651 (JP)**

• **NAKADA, Shigeyuki**
**Tamano-shi**
**Okayama 706-8651 (JP)**
• **HOSHISHIMA, Kazuteru**
**Tamano-shi**
**Okayama 706-8651 (JP)**
• **DOI, Kyouji**
**Tamano-shi**
**Okayama 706-8651 (JP)**

(74) Representative: **Hards, Andrew**
**Global IP Europe**
**Patentanwaltskanzlei**
**Prinzregentenstraße 11a**
**80538 München (DE)**

(54) **FLUORESCENCE DETECTION APPARATUS, FLUORESCENCE DETECTION METHOD, AND METHOD FOR PROCESSING FLUORESCENCE SIGNAL**

(57) Disclosed herein is a fluorescence detecting apparatus. The fluorescence detecting apparatus includes a light-receiving element that receives fluorescence emitted from an object to be measured irradiated with laser light modulated at a predetermined frequency and outputs a fluorescence signal at an adjusted output level; a signal processing unit that mixes the outputted fluorescence signal and a modulation signal with the frequency to generate fluorescence data including information about phase and intensity; and an analyzing device that calculates a first phase shift of the fluorescence emitted from the object to be measured with respect to the modulation signal, calculates a second phase shift by correcting the calculated first phase shift depending on conditions for adjusting the output level, and calculates a fluorescence relaxation time of the fluorescence emitted from the object to be measured using the calculated second phase shift.

FIG.6

EP 2 522 987 A1

**Description**

Technical Field

The present invention relates to a fluorescence detecting apparatus and a fluorescence detecting method, by which a fluorescence signal of fluorescence emitted from an object to be measured irradiated with laser light is processed, and a fluorescence signal processing method.

Background Art

In medical and biological fields, flow cytometers are widely used. A flow cytometer uses a photoelectric converter, such as a photomultiplier tube or an avalanche photodiode, that receives fluorescence emitted from objects to be measured, such as cells or genes, irradiated with laser light to determine the kind of object to be measured and the number of the objects to be measured and further analyze the characteristics of each of the objects to be measured.

Specifically, in such a flow cytometer, objects to be measured obtained by labeling objects to be analyzed such as biological substances (e.g., cells, DNA, RNA, enzymes, proteins) with a fluorochrome are allowed to flow in a sheath fluid flowing in a tube under pressure at a speed of about 10 m/s or less to form a laminar sheath flow. The flow cytometer irradiates the objects to be measured in the flow with laser light, receives fluorescence emitted from the fluorochrome attached to the objects to be analyzed, and identifies the objects to be analyzed by using the fluorescence as a label for identifying the objects to be analyzed.

The flow cytometer can measure, for example, the intracellular relative amounts of DNA, RNA, enzymes, proteins, etc. in a cell and analyze their activities in a short period of time. Further, the flow cytometer identifies predetermined types of cells or chromosomes based on fluorescence and selectively collects only the identified cells or chromosomes in a living state in a short period of time with the use of a cell sorter or the like.

When the flow cytometer is used, a larger number of objects to be measured are required to be accurately identified in a shorter period of time from information about fluorescence.

For example, a fluorescence detecting apparatus and a fluorescence detecting method are known which are capable of accurately identifying many kinds of objects to be measured in a short period of time by calculating the fluorescence lifetime (fluorescence relaxation time) of fluorescence emitted from a fluorochrome in the objects to be measured irradiated with laser light (Patent Literature 1).

In such a fluorescence detecting apparatus, an object to be measured is irradiated with laser light whose intensity is modulated, fluorescence emitted from the object to be measured is received by a photoelectric converter, the phase shift of a fluorescence signal outputted from the photoelectric converter with respect to a modulation signal used to modulate the intensity of laser light is determined, and a fluorescence relaxation time is calculated from the phase shift.

Citation List

Patent Literature

Patent Literature 1: JP-A-2006-226698

Summary of Invention

Technical Problem

The phase shift used to calculate a fluorescence relaxation time is calculated by eliminating the influence of a phase shift caused by the characteristics of the photoelectric converter itself that receives fluorescence and eliminating the influence of a phase shift caused by the optical path lengths of laser light and of fluorescence and the length of an electrical circuit in the apparatus. However, the phase shift used to calculate a fluorescence relaxation time slightly changes depending on the output level of the fluorescence signal outputted from the photoelectric converter, and therefore a fluorescence relaxation time is not always calculated accurately. Particularly, calculation of an accurate fluorescence relaxation time is required to, for example, determine the presence or absence of FRET (Fluorescence Resonance Energy Transfer).

In order to solve the above problem, it is an object of the present invention to provide a fluorescence detecting apparatus and a fluorescence detecting method, which are capable of calculating an accurate fluorescence relaxation time when a fluorescence signal of fluorescence emitted from an object to be measured irradiated with laser light is processed, and a fluorescence signal processing method for use in the fluorescence detecting apparatus and the fluorescence detecting method.

An aspect of the invention is a fluorescence detecting apparatus for processing a fluorescence signal of fluorescence emitted from an object to be measured irradiated with laser light. The apparatus includes:

a light source unit for emitting laser light whose intensity is modulated using a modulation signal with a predetermined frequency;
a light-receiving unit provided with an output level-adjustable light-receiving element for receiving fluorescence emitted from an object to be measured irradiated with the laser light and for outputting a fluorescence signal at an adjusted output level;
a first processing unit for mixing the fluorescence signal and the modulation signal with the frequency to generate fluorescence data including information about phase and intensity; and

a second processing unit for calculating a first phase shift of the fluorescence with respect to the modulation signal using the fluorescence data, calculating a second phase shift by making a correction to the calculated first phase shift depending on conditions for adjusting the output level, and calculating a fluorescence relaxation time of the fluorescence using the calculated second phase shift.

The light-receiving unit preferably includes an adjusting section for adjusting the output level using an adjustment parameter value given to the light-receiving element.

Then, the second processing unit is preferably configured to use a correction formula determined depending on the adjustment parameter value for making the correction. Further preferably, the second processing unit is configured to previously store a value of a constant used in the correction formula and to call and use the value of the constant for making the correction.

The second processing unit is preferably provided with an output level adjusting section for extracting the value of the constant used in the correction formula when a relationship between the adjustment parameter value and the output level indicates that the output level of the light-receiving element is not saturated. The relationship is determined by changing, in advance, the adjustment parameter value given to the light-receiving element.

When a reference object having a known fluorescence relaxation time is used as the object to be measured, the output level adjusting section is preferably configured to extract the value of the constant so that the fluorescence relaxation time calculated from the second phase shift approximates the known fluorescence relaxation time of the reference object by the correction.

For example, the light-receiving element may be a photomultiplier tube and the adjustment parameter value is a voltage applied to an electrode of the photomultiplier tube.

Then, the correction formula is preferably represented by the following formula: $\theta_\tau = X - \theta_{meas} - A \cdot V^{(-1/2)}$, where X and A are constants. The voltage applied to the electrode is represented by V, and the first phase shift is represented by $\theta_{meas}$, and the second phase shift is represented by $\theta_{\tau'}$.

An adjustable neutral density filter is preferred to be provided in front of a light-receiving surface of the light-receiving element.

Other aspect of the invention is a fluorescence detecting method for use in a fluorescence detecting apparatus for processing a fluorescence signal of fluorescence emitted from an obj ect to be measured irradiated with laser light. The method includes the steps of:

emitting laser light whose intensity is modulated using a modulation signal with a predetermined frequency;

receiving by a light-receiving element fluorescence emitted from an object to be measured irradiated with the laser light and outputting a fluorescence signal at an adjusted output level;

mixing the fluorescence signal and the modulation signal with a predetermined frequency to generate fluorescence data including information about phase and intensity; and

calculating a first phase shift of the fluorescence with respect to the modulation signal using the fluorescence data, calculating a second phase shift by making a correction to the calculated first phase shift depending on conditions for adjusting the output level, and calculating a fluorescence relaxation time of the fluorescence using the calculated second phase shift.

Other aspect of the invention is a method for allowing a fluorescence detecting apparatus to process a fluorescence signal of fluorescence emitted from a reference object having a known fluorescence relaxation time by irradiation with laser light. The method includes:

a first step of emitting laser light whose intensity is modulated using a modulation signal with a predetermined frequency;

a second step of receiving by a light-receiving element fluorescence emitted from a reference object irradiated with the laser light and output a fluorescence signal at an adjusted output level;

a third step of mixing the fluorescence signal and the modulation signal with a predetermined frequency to generate fluorescence data including information about phase and intensity;

a fourth step of calculating a first phase shift of the fluorescence with respect to the modulation signal using the fluorescence data and storing a pair of the calculated first phase shift and an adjustment parameter value given to the light-receiving element to adjust the output level;

a fifth step of storing two or more pairs of the first phase shift and the adjustment parameter value by repeating the first to fourth steps while varying the adjustment parameter value;

a sixth step of calling the two or more stored pairs and approximating a regression formula expressing a relationship between the adjustment parameter value and the first phase shift to a relationship represented by the two or more called pairs, to extract a value of an unknown constant included in the regression formula; and

a seventh step of storing the extracted value of the constant.

The adjustment parameter value is preferably changed so that the output level is reduced every time the first to fourth steps are repeated.

The fluorescence to be received by the light-receiving element is preferably attenuated by a neutral density filter before being received by the light-receiving element so that the output level of the fluorescence signal outputted by the light-receiving element is not saturated. Advantageous Effects of Invention

The fluorescence detecting apparatus and the fluorescence detecting method according to the above aspects of the present invention make it possible to calculate an accurate fluorescence relaxation time when a fluorescence signal of fluorescence emitted from an object to be measured irradiated with laser light is processed.

Further, the fluorescence signal processing method according to the above aspect of the present invention makes it possible to efficiently determine the value of a constant for use in a correction formula for calculating an accurate fluorescence relaxation time.

Brief Description of Drawings

Fig. 1 is a schematic diagram illustrating the configuration of a flow cytometer using a fluorescence detecting apparatus according to an embodiment of the present invention.

Fig. 2 is a diagram illustrating one example of the configuration of a laser light source unit used in the flow cytometer illustrated in Fig. 1.

Fig. 3 is a schematic diagram illustrating the configuration of one example of a light-receiving unit used in the flow cytometer illustrated in Fig. 1.

Fig. 4 is a schematic diagram illustrating the configuration of a photomultiplier tube used in the flow cytometer illustrated in Fig. 1.

Fig. 5 is a schematic diagram illustrating the configuration of a control-processing unit used in the flow cytometer illustrated in Fig. 1.

Fig. 6 is a schematic diagram illustrating the configuration of an analyzing device used in the flow cytometer illustrated in Fig. 1.

Figs. 7A and 7B are graphs illustrating the relationship between a control voltage applied to the photomultiplier tube illustrated in Fig. 4 and the phase shift angle $\theta_{meas}$ of fluorescence.

Fig. 8 is a flow chart of an example of the process of determining the value of a constant X and the value of a constant A for use in a correction formula by using the flow cytometer illustrated in Fig. 1.

Fig. 9 is a diagram for explaining an example of saturation of the output level of a fluorescence signal outputted by the photomultiplier tube illustrated in Fig. 4.

Description of Embodiment

Hereinbelow, a fluorescence detecting apparatus, a fluorescence detecting method, and a fluorescence signal processing method according to the present invention will be described in detail.

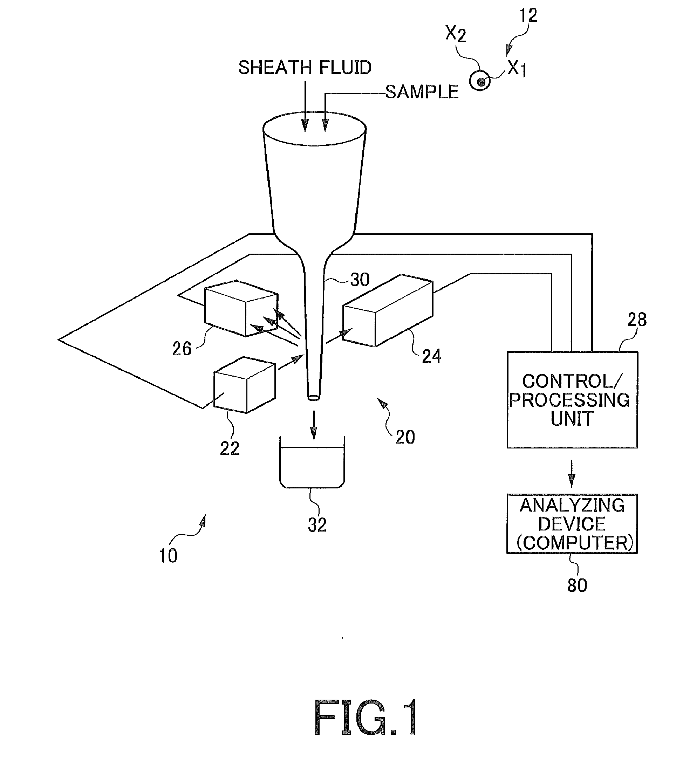

Fig. 1 is a schematic diagram illustrating the configuration of a flow cytometer 10 using a fluorescence detecting apparatus according to an embodiment of the present invention.

The flow cytometer 10 includes a signal processing device 20 and an analyzing device (computer) 80. The signal processing device 20 detects and processes a fluorescence signal of fluorescence emitted from a sample 12 as an object to be measured when the sample 12 is irradiated with laser light. The analyzing device (computer) 80 calculates a fluorescence intensity and a fluorescence relaxation time from a processing result obtained by the signal processing device 20. Hereinbelow, the flow cytometer 10 will be described with reference to a case where the sample 12 used as an object to be measured is a cell (object to be analyzed) $X_2$ labeled with a fluorescent protein (fluorochrome) $X_1$. The fluorochrome used in this embodiment may be one other than a fluorescent protein. The object to be analyzed used in this embodiment may be one other than a cell, and examples thereof include biological substances such as DNA, RNA, enzymes, and proteins and artificially-produced microbeads. It is to be noted that the sample 12 illustrated in Fig. 1 has a structure in which, for example, the fluorescent proteins $X_1$, entering the cell $X_2$, are introduced into and dispersed in the cell $X_2$. The number of fluorescent proteins $X_1$ to be attached to the cell $X_2$ is not limited to one and may be two or more.

The signal processing device 20 has a laser light source unit 22, light-receiving units 24 and 26, a control-processing unit 28, and a tube 30. The control-processing unit 28 has a control unit that modulates the intensity of laser light emitted from the laser light source unit 22 at a predetermined frequency and a signal processing unit that processes a fluorescence signal of fluorescence emitted from the sample 12. The tube 30 allows the samples 12 to flow in a high-speed flow of sheath fluid to produce a flow of the samples 12.

A recovery container 32 is provided at the outlet of the tube 30. The flow cytometer 10 may include a cell sorter for separating biological substances such as predetermined cells in the samples 12 in a short period of time by irradiation with laser light to sort biological substances into different recovery containers.

The laser light source unit 22 emits laser light with a predetermined wavelength. A lens system is provided to focus the laser light on a predetermined position in the tube 30, and a measurement point of the sample 12 is provided at this focus position.

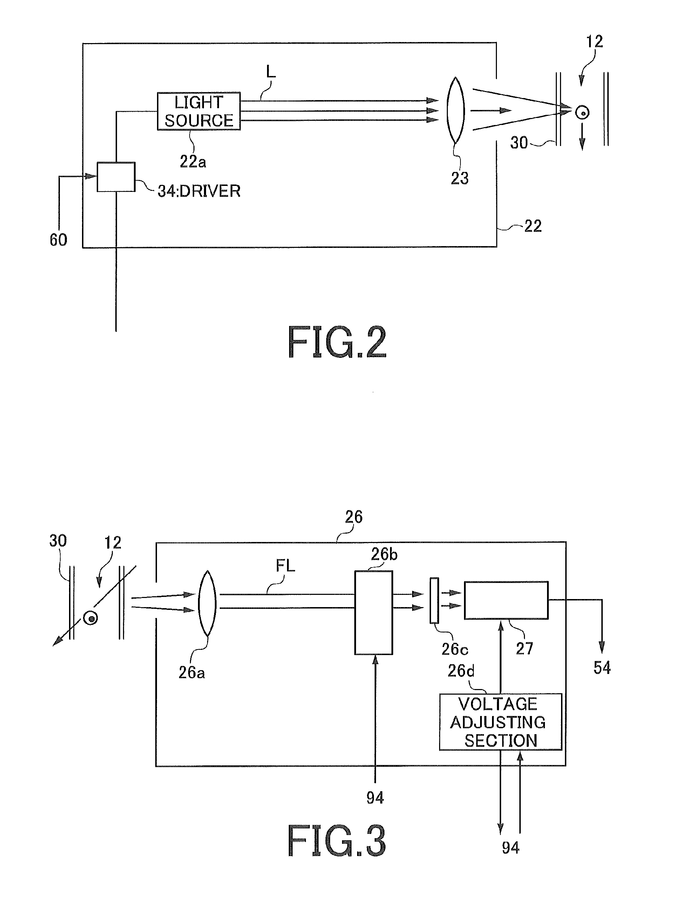

Fig. 2 is a diagram illustrating one example of the configuration of the laser light source unit 22.

The laser light source unit 22 emits intensity-modulated laser light with a wavelength in the visible light range. The laser light source unit 22 has a light source 22a. The light source 22a emits, as CW (continuous wave) laser light, laser light L with a wavelength that excites the fluorescent protein $X_1$ while modulating the intensity of the laser light L using a modulation signal with a predetermined frequency f.

Further, the laser light source unit 22 has a lens system 23 and a laser driver 34. The lens system 23 focuses the laser light L onto the center of the tube 30 to provide a measurement point. The laser driver 34 drives the light source 22a.

As the light source that emits the laser light L, for example, a semiconductor laser is employed. The output power of the laser light L is, for example, about 5 to 100 mW.

On the other hand, the frequency (modulation frequency) f used to modulate the intensity of the laser light L has a modulation period slightly longer than a fluorescence relaxation time and is, for example, 10 to 50 MHz.

The light source 22a oscillates at a predetermined wavelength band so that the fluorochrome is excited by the laser light L and emits fluorescence of a specific wavelength band. The sample 12 to be excited by the laser light L is irradiated with the laser light L at the measurement point in the tube 30 when passing through the measurement point. At this time, the fluorescent protein $X_1$ emits fluorescence at a specific wavelength.

The light-receiving unit 24 and the laser light source unit 22 are arranged on opposite sides of the tube 30. The light-receiving unit 24 has a photoelectric converter that receives the laser light forward-scattered by the sample 12 passing through the measurement point and outputs a detection signal of the sample 12 to announce that the sample 12 passes through the measurement point. The detection signal outputted from the light-receiving unit 24 is supplied to the control·processing unit 28 and the analyzing device 80 and used as a trigger signal that announces timing, at which the sample 12 passes through the measurement point in the tube 30, to start processing, an ON signal for starting processing, and an OFF signal.

On the other hand, the light-receiving unit 26 is arranged in a direction perpendicular to both a direction in which the laser light emitted from the laser light source unit 22 travels and a direction in which the sample 12 moves in the tube 30. The light-receiving unit 26 has a photomultiplier tube that receives fluorescence emitted from the sample 12 irradiated with the laser light at the measurement point. In this embodiment, a well-known photoelectric converter such as an avalanche photodiode may be employed instead of the photomultiplier tube. Fig. 3 is a schematic diagram illustrating the configuration of one example of the light-receiving unit 26.

The light-receiving unit 26 illustrated in Fig. 3 has a lens system 26a that focuses a fluorescence signal from the sample 12, an ND filter 26b, a bandpass filter 26c, a voltage adjusting section 26d, and a photomultiplier tube (light-receiving element) 27.

The lens system 26a is configured to focus fluorescence that has entered the light-receiving unit 26 onto a light-receiving surface of the photomultiplier tube 27.

The ND filter 26b attenuates fluorescence that has entered the light-receiving unit 26. As will be described later, the light-receiving unit 26 uses the ND filter 26b to attenuate fluorescence to prevent the output level of a fluorescence signal from being saturated even when a control voltage applied to an anode of the photomultiplier tube 27 is maximized.

The bandpass filter 26c is a filter that is provided in front of the light-receiving surface of the photomultiplier tube 27 and transmits only fluorescence of a predetermined wavelength band.

The photomultiplier tube 27 is a light-receiving element that converts light received by its photoelectric surface into an electrical signal and outputs a fluorescence signal. The output level of the fluorescence signal outputted by the photomultiplier tube 27 is adjusted. Fluorescence received by the photomultiplier tube 27 is derived from the laser light whose intensity is modulated at a predetermined frequency, and therefore the intensity of the outputted fluorescence signal also varies at the predetermined frequency. The fluorescence signal outputted by receiving fluorescence is supplied to the control·processing unit 28.

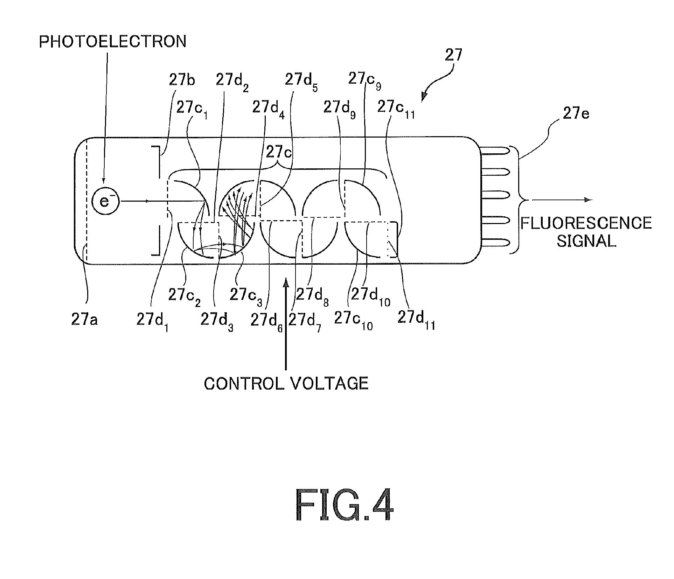

Fig. 4 is a schematic diagram illustrating the configuration of the photomultiplier tube 27. The photomultiplier tube 27 has a vacuum vessel in which a vacuum atmosphere of, for example, about $10^{-4}$ Pa is kept, and in the vacuum vessel, a cathode 27a having a light-receiving surface as its surface, a focusing electrode 27b, a photomultiplier section 27c, and anodes $27d_1$ to $27d_{11}$ are provided. Further, the photomultiplier tube 27 has a stem 27e outside the vacuum vessel.

When light enters the cathode 27a, photoelectrons are emitted from a photoelectric surface of the cathode 27a into a vacuum within the vacuum vessel. The photoelectrons are directed by the focusing electrode 27b toward the photomultiplier section 27c. The photoelectrons are attracted to the anode $27d_1$ and collide with a dynode $27c_1$ of the photomultiplier section 27c, and the dynode $27c_1$ emits secondary electrons. Further, the secondary electrons emitted from the dynode $27c_1$ are attracted to the anode $27d_2$ provided in front of a dynode $27c_2$ and collide with the dynode $27c_2$, and the dynode $27c_2$ further emits secondary electrons. In this way, the electrons, being attracted by the anode $27d_n$ (n is an integer of 2 or more but 10 or less), collide with dynodes $27c_3$ to $27c_{10}$, and sequential emission of secondary electrons from the dynodes $27c_3$ to $27c_{10}$, whereby the electrons are multiplied. The multiplied electrons are attracted by the anode $27d_{11}$ and collected at a dynode $27c_{11}$, and a fluorescence current is produced through the stem 27e.

At this time, the electron transfer rate of the electrons is determined by a voltage applied to the anodes $27d_1$ to $27d_{11}$, and the energy of electrons colliding with the dynodes $27c_1$ to $27c_{10}$ is determined by the electron transfer rate. Therefore, the amount of secondary electrons emit-

ted from the dynodes $27c_1$ to $27c_{10}$ is adjusted by varying a voltage applied to the anodes $27d_1$ to $27d_{11}$. The flow cytometer 10 uses, as a control voltage for controlling the output level of the fluorescence signal (fluorescence current), a voltage applied to the anodes $27d_1$ to $27d_{11}$.

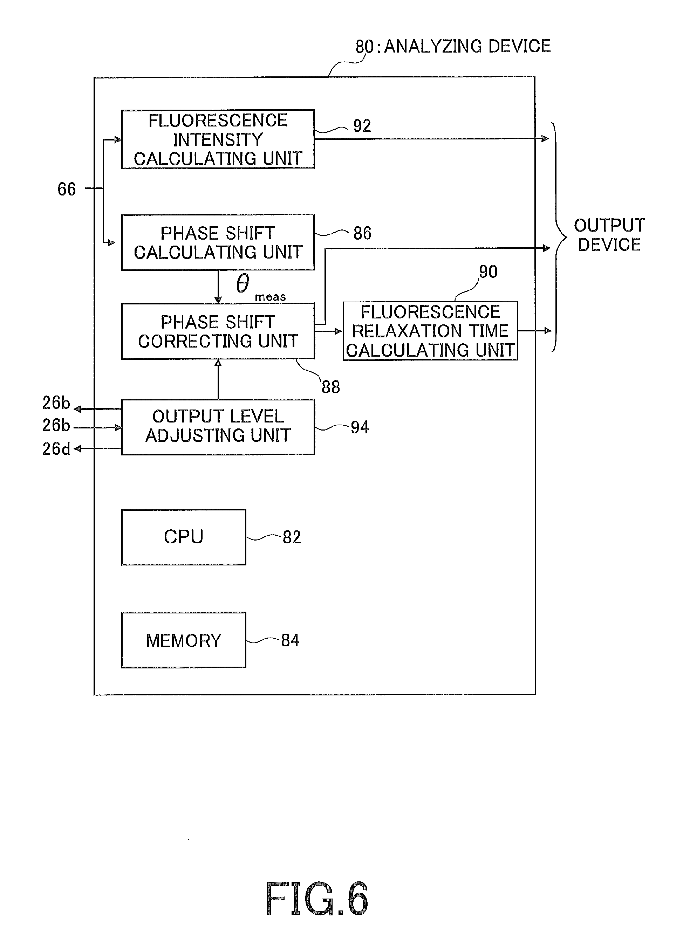

The voltage adjusting section 26d (see Fig. 3) sets the control voltage for controlling the output level of the fluorescence signal and applies the control voltage to the photomultiplier tube 27. The voltage adjusting section 26d sets the control voltage under the instruction of an output level adjusting unit 94 of the analyzing device 80 that will be described later. The voltage adjusting section 26d provides, to a phase shift correcting unit 88 (see Fig. 6) that will be described later, information about the control voltage applied to the photomultiplier tube 27.

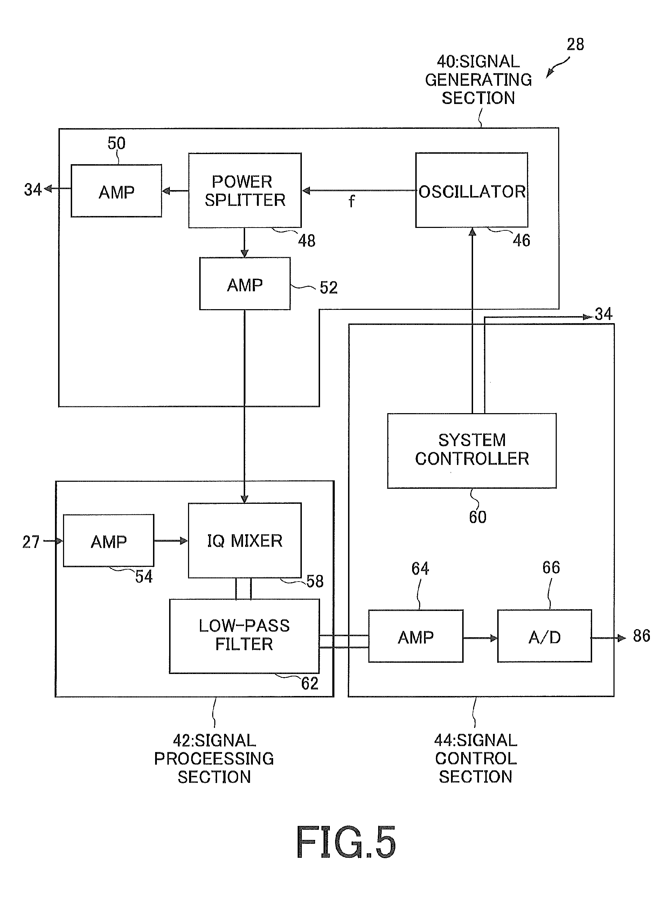

As illustrated in Fig. 5, the control·processing unit 28 has a signal generating section 40, a signal processing section 42, and a signal control section 44. The signal generating section 40 generates a modulation signal for modulating the intensity of the laser light L at a predetermined frequency f.

Specifically, the signal generating section 40 has an oscillator 46, a power splitter 48, and amplifiers 50 and 52. The signal generating section 40 supplies the generated modulation signal to the laser driver 34 of the laser light source unit 22 and to the signal processing section 42. As will be described later, the reason why the modulation signal is supplied to the signal processing section 42 is that the modulation signal is used as a reference signal for detecting the fluorescence signal outputted from the photomultiplier tube 27. It is to be noted that the modulation signal is a signal obtained by adding a sinusoidal signal of a predetermined frequency to a DC signal component. The frequency is set to a value in the range of 10 to 50 MHz.

The signal processing section 42 extracts information about the phase shift of fluorescence emitted by irradiation with the laser light with the use of the fluorescence signal outputted from the photomultiplier tube 27. The signal processing section 42 has an amplifier 54, an IQ mixer 58, and a low-pass filter 62. The amplifier 54 amplifies the fluorescence signal outputted from the photomultiplier tube 27.

The IQ mixer 58 is a device that mixes the fluorescence signal supplied from the photomultiplier tube 27 and the modulation signal supplied from the signal generating section 40 as a reference signal. Specifically, the IQ mixer 58 multiplies the reference signal and the fluorescence signal (RF signal) to generate an I signal of the fluorescence signal, which contains a component in phase with the modulation signal, and a Q signal of the fluorescence signal, which contains a 90 degrees phase-shifted component with respect to the modulation signal. The I signal containing a component in phase with the modulation signal is generated by mixing the modulation signal and the fluorescence signal, and the Q signal containing a 90 degrees phase-shifted component with respect to the modulation signal is generated by mixing the modulation signal whose phase is shifted by 90° and the fluorescence signal.

The low-pass filter 62 filters a low-frequency signal of the I signal generated by the IQ mixer 58 and a low-frequency signal of the Q signal generated by the IQ mixer 58. By performing such filtering, the component (Re component) of the fluorescence signal, which is in phase with the modulation signal, and the component (Im component) of the fluorescence signal, which is phase-shifted by 90 degrees with respect to the modulation signal, are extracted as fluorescence data. The extracted components are sent to the signal control section 44. Hereinafter, a process including mixing by the IQ mixer 58 and filtering by the low-pass filter 62 is referred to as a frequency down conversion process, and data obtained by this process is referred to as fluorescence data.

The signal control section 44 amplifies the Re component and the Im component of the fluorescence signal sent from the signal processing section 42, and performs AD conversion.

Specifically, the signal control section 44 has a system controller 60 that provides directions for controlling the operation of each of the units and manages all the operations of the flow cytometer 10, an amplifier 64 that amplifies the Re component and the Im component generated by the signal processing section 42, and an A/D converter 66 that samples the amplified Re component and Im component. The amplifier 64 and the A/D converter 66 may be operated in the analyzing device 80 that will be described later.

The analyzing device 80 determines the phase shift angle of fluorescence with respect to the laser light from the Re component and the Im component A/D converted by the signal control section 44, and further determines a fluorescence relaxation time constant (fluorescence relaxation time) from the phase shift angle. Further, the analyzing device 80 determines a fluorescence intensity from the Re component and the Im component. Fig. 6 is a schematic diagram illustrating the configuration of the analyzing device 80.

The analyzing device 80 is constituted from a computer including a CPU 82 and a memory 84. The analyzing device 80 further includes a phase shift calculating unit 86, a phase shift correcting unit 88, a fluorescence relaxation time calculating unit 90, a fluorescence intensity calculating unit 92, and an output level adjusting unit 94. Each of the units is a software module that performs its function by executing software on the computer. These units may be, of course, implemented by dedicated circuits.

The phase shift calculating unit 86 calculates, from the fluorescence data of fluorescence emitted from the fluorescent protein $X_1$, the argument of a complex number whose real part is the Re component and imaginary part is the Im component ($\tan^{-1}$ (Im component of fluorescence data/Re component of fluorescence data)) as a phase shift angle $\theta_{meas}$. The phase shift calculating unit 86 provides the phase shift angle $\theta_{meas}$ to the phase

shift correcting unit 88.

The phase shift correcting unit 88 calculates a phase shift angle $\theta_\tau$ derived from fluorescence emitted from the fluorescent protein $X_1$ by correcting the phase shift angle $\theta_{meas}$ calculated by the phase shift calculating unit 86. The correction is based on the control voltage (adjustment parameter value) applied to the anodes $27d_1$ to $27d_{11}$ of the photomultiplier tube 27, that is, the correction is based on conditions for adjusting the output level of the fluorescence signal performed by the voltage adjusting section 26d.

This correction is performed to prevent a change in the phase shift angle $\theta$ caused by a change in the transfer rate of photoelectrons and secondary electrons in the photomultiplier tube 27 to calculate an accurate fluorescence relaxation time. In the photomultiplier tube 27, the transfer rate of photoelectrons and secondary electrons varies depending on the control voltage. Therefore, the phase shift angle $\theta_{meas}$ is corrected depending on the set control voltage.

When the length between a dynode that produces secondary electrons and an anode that attracts secondary electrons in the photomultiplier tube 27 is represented by 1, the time t required for a photoelectron and a secondary electron to travel the length 1 is represented by the following formula (1):

$$ t = (2ml^2/V/q)^{(1/2)} \quad (1) $$

wherein m is the mass of an electron, q is the amount of charge of an electron, and V is the control voltage.

On the other hand, the phase shift angle $\theta$ is proportional to the time t. Therefore, a phase shift angle (a phase shift angle derived from the photomultiplier tube 27) $\theta_{pmt}$ resulting from the time required for an electron to travel in the photomultiplier tube 27 is determined by the addition of a constant B and represented by the formula: $\theta_{pmt} = A \cdot V^{(-1/2)} + B$, wherein A and B are constants.

On the other hand, the phase shift angle $\theta_{meas}$ calculated by the phase shift calculating unit 86 can be represented by the following formula (2):

$$ \theta_{meas} = \theta_0 - (\theta_{pmt} + \theta_d + \theta_\tau) \quad (2) $$

wherein $\theta_0$ is a reference phase shift angle (which is a constant), $\theta_{pmt}$ is a phase shift angle derived from the photomultiplier tube 27, $\theta_d$ is a phase shift angle derived from the total path length of the laser light, the path length of fluorescence, and the electrical circuit length of the fluorescence signal in the flow cytometer 10, and $\theta_\tau$ is a phase shift angle derived from fluorescence emitted from the fluorescent protein $X_1$.

Therefore, the phase shift angle $\theta_\tau$ derived from fluorescence emitted from the fluorescent protein $X_1$ is represented by the following formula (3):

$$ \theta_\tau = \theta_0 - (\theta_{pmt} + \theta_d + \theta_{meas}) \quad (3) $$

Substitution of $\theta_{pmt} = A \cdot V^{(-1/2)} + B$ into the above formula (3) yields the following formula (4):

$$ \theta_\tau = X - \theta_{meas} - A \cdot V^{(-1/2)} \quad (4) $$

wherein X is represented as $X = \theta_0 - B - \theta_d$ and is a constant independent of the control voltage.

The memory 84 of the analyzing device 80 previously stores the value of the constant X and the value of the constant A. The phase shift correcting unit 88 calls the values of the constant X and the constant A from the memory 84 and gives them to the formula (4) to prepare a correction formula. Then, the phase shift correcting unit 88 calculates, with the use of the prepared correction formula, the phase shift angle $\theta_\tau$ from the phase shift angle $\theta_{meas}$ provided by the phase shift calculating unit 86 and the control voltage V provided by the voltage adjusting section 26d.

The phase shift correcting unit 88 provides the calculated phase shift angle $\theta_\tau$ to the fluorescence relaxation time calculating unit 90.

The fluorescence relaxation time calculating unit 90 calculates the fluorescence relaxation time $\tau$ of the fluorescent protein $X_1$ according to the formula $\tau = 1/(2\pi f) \cdot \tan(\theta_\tau)$, using the phase shift angle $\theta_\tau$ calculated by the phase shift correcting unit 88. Here, f is a frequency used to modulate the intensity of the laser light L. The reason why the fluorescence relaxation time $\tau$ can be calculated according to the formula $\tau = 1/(2\pi f) \cdot \tan(\theta_\tau)$ is that a fluorescence phenomenon shows a change in accordance with a primary relaxation process.

The fluorescence intensity calculating unit 92 determines the absolute value of a complex number whose real part is the Re component and imaginary part is the Im component from the fluorescence data sent from the AD converter 66 to calculate the fluorescence intensity of fluorescence emitted from the fluorescent protein $X_1$.

The calculated fluorescence intensity, phase shift angle $\theta_\tau$, and fluorescence relaxation time $\tau$ of the fluorescent protein $X_1$ are outputted as result information to an output device (not shown) such as a printer or a display. Further, the result information is used for a statistical processing as a measurement result obtained every time the sample 12 passes through the measurement point in the tube 30. The output level adjusting unit 94 generates a control signal for setting the control voltage applied to the photomultiplier tube 27. The control voltage is arbitrarily set without saturating the output level of the fluorescence signal outputted from the photomultiplier tube 27. The output level adjusting unit 94 has at least information about the current control voltage applied to the photomultiplier tube 27. Further, the output level adjusting unit 94 performs calibration that will be described later to calculate the value of the constant X and the value of the constant A.

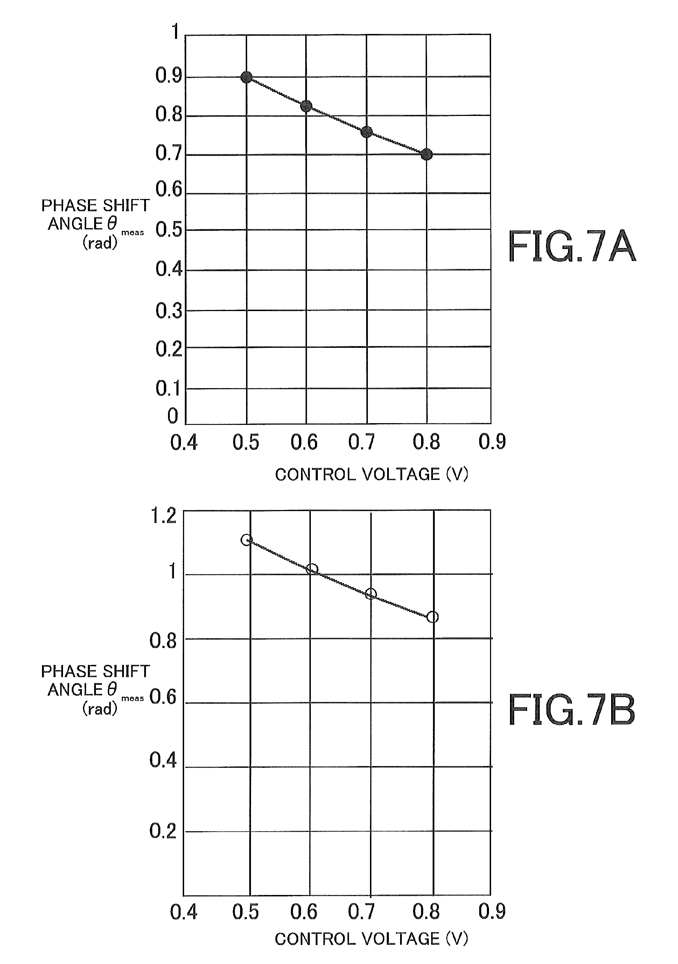

Fig. 7A is a graph illustrating a comparison of the phase shift angles $\theta_{meas}$ of fluorescence of a fluorescent protein Qdot555. The comparison is made between a phase shift angle $\theta_{meas}$ which was determined by calculation using the prepared correction formula, to which the value of the constant X and the value of the constant A were given, and a phase shift angle $\theta_{meas}$ which was measured using the flow cytometer 10 and calculated by the analyzing device 80. Fig. 7B is a graph illustrating another comparison of the phase shift angles $\theta_{meas}$ of fluorescence of a fluorescent protein PerCP. The comparison is made between a phase shift angle $\theta_{meas}$ which was determined by calculation using the prepared correction formula, to which the value of the constant X and the value of the constant A were given, and a phase shift angle $\theta_{meas}$ which was measured using the flow cytometer 10 and calculated by the analyzing device 80. Here, since each of the fluorescent proteins Qdot555 and PerCP has a known fluorescence relaxation time $\tau$, a value calculated by the formula $\theta_{\tau} = \tan^{-1}(2\pi f\tau)$ was used as the phase shift angle $\theta_{\tau}$. In each of Figs. 7A and 7B, the curve line represents the results of calculation of the phase shift angle $\theta_{meas}$ using the correction formula and the symbol "●" represents the results of measurement of the phase shift angle $\theta_{meas}$.

As can be seen from Figs. 7A and 7B, the results of calculation by using the correction formula extremely agree with the results of measurement. Therefore, correction using the correction formula, that is, the phase shift angle $\theta_{\tau}$ calculated by the formula (4) makes it possible to calculate an accurate fluorescence relaxation time.

Hereinbelow, a fluorescence detecting method used in the flow cytometer 10 will be described.

First, in the flow cytometer 10, the laser light source unit 22 emits, as irradiation light, laser light whose intensity is modulated at a predetermined frequency f.

Then, the light-receiving unit 26 receives fluorescence and outputs a fluorescence signal.

The control·processing unit 28 mixes the outputted fluorescence signal and a modulation signal for modulating the intensity of laser light L to generate fluorescence data including the phase shift angle of the fluorescence signal with respect to the modulation signal and intensity amplitude.

The phase shift calculating unit 86 of the analyzing device 80 calculates a phase shift angle $\theta_{meas}$ from the generated fluorescence data. The phase shift correcting unit 88 calculates a phase shift angle $\theta_{\tau}$ from the calculated phase shift angle $\theta_{meas}$ according to the correction formula (4) to which the value of the constant X and the value of the constant A are given. Further, the fluorescence relaxation time calculating unit 90 calculates the

fluorescence relaxation time $\tau$ of the fluorescent protein $X_1$ using the phase shift angle $\theta_{\tau}$ according to the formula: $\tau = \tan(\theta_{\tau})/(2\pi f)$. On the other hand, the fluorescence intensity calculating unit 92 calculates a fluorescence intensity by determining the absolute value of the generated fluorescence data.

The calculated fluorescence relaxation time, phase shift angle $\theta_{\tau}$, and fluorescence intensity are outputted from an output device not shown.

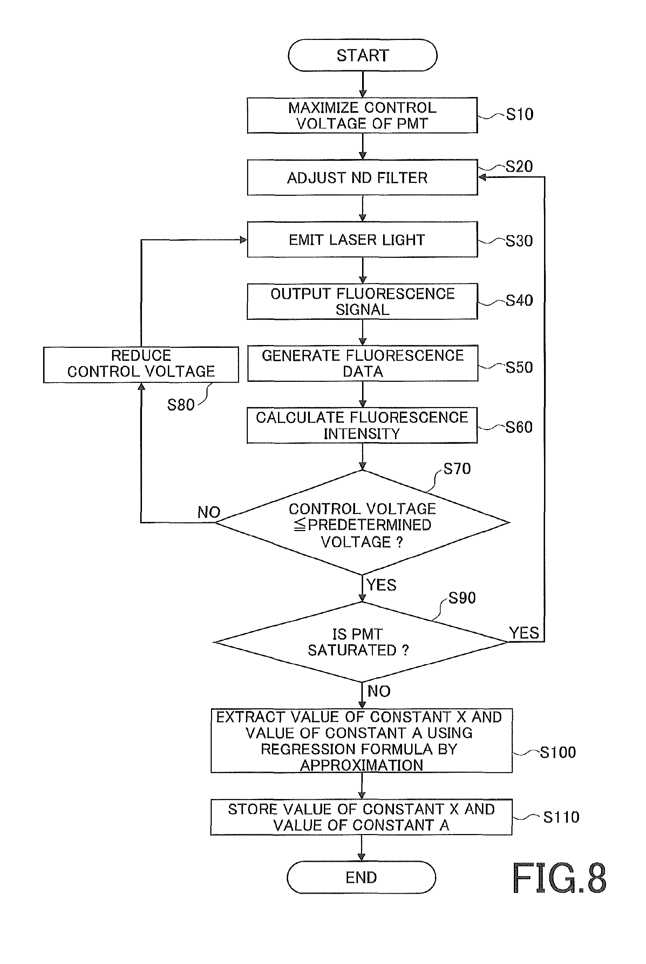

Fig. 8 is a flow chart of an example of calibration for determining the value of the constant X and the value of the constant A to be stored in the memory 84 with the use of the flow cytometer 10. When the value of the constant X and the value of the constant A are determined, a fluorescent protein having a known fluorescence relaxation time $\tau$ is used as the fluorescent protein $X_1$ for measurement using the flow cytometer 10.

First, the output level adjusting unit 94 of the analyzing device 80 maximizes the control voltage V of the photomultiplier tube (hereinafter, referred to as "PMT") 27 (Step S10). The maximization of the control voltage V is performed to achieve highly-sensitive detection of fluorescence even when the fluorescence is very weak. The control voltage is adjusted by the voltage adjusting section 26d.

Then, the output level adjusting unit 94 of the analyzing device 80 appropriately adjusts the ND filter 26b (Step S20). Attenuation of fluorescence by the ND filter is performed to prevent the output level of a fluorescence signal outputted from the PMT 27, which depends on the amount of light, from being saturated when the control voltage of the PMT 27 is maximized to maximize the sensitivity of the PMT 27. The ND filter 26b is set to, for example, intermediate level between minimum and maximum attenuation levels.

Then, the light source 22a emits laser light L whose intensity is modulated at a frequency f (Step S30). Then, the PMT 27 receives fluorescence emitted from the sample 12 irradiated with the laser light L when the sample 12 passes through the measurement point. Then, the PMT 27 outputs a fluorescence signal at an output level adjusted by the control voltage V (Step S40). The fluorescence signal is processed by the signal processing section 42 and the signal control section 44 so that fluorescence data including an Re component and an Im component is generated (Step S50). Then, the fluorescence intensity calculating unit 92 of the analyzing device 80 calculates a fluorescence intensity from the fluorescence data (Step S60). The fluorescence intensity is expressed as the absolute value of the fluorescence data.

Then, the output level adjusting unit 94 determines whether or not the set control voltage V is equal to or less than a predetermined voltage (Step S70).

When the determination result is negative, the output level adjusting unit 94 allows the voltage adjusting section 26d to reduce the control voltage V by a certain amount (Step S80). Then, the steps from Step S30 to Step S70 are repeated using the reduced control voltage V. That is, the control voltage V is adjusted in such a manner that the level of the fluorescence signal is reduced every time the steps from Step S30 to Step S70 are repeated. When the determination result in Step S70 is positive, the output level adjusting unit 94 further determines whether the fluorescence signal outputted from the PMT 27 is saturated or not using the change of the fluorescence intensity with respect to the control voltage V (Step S90).

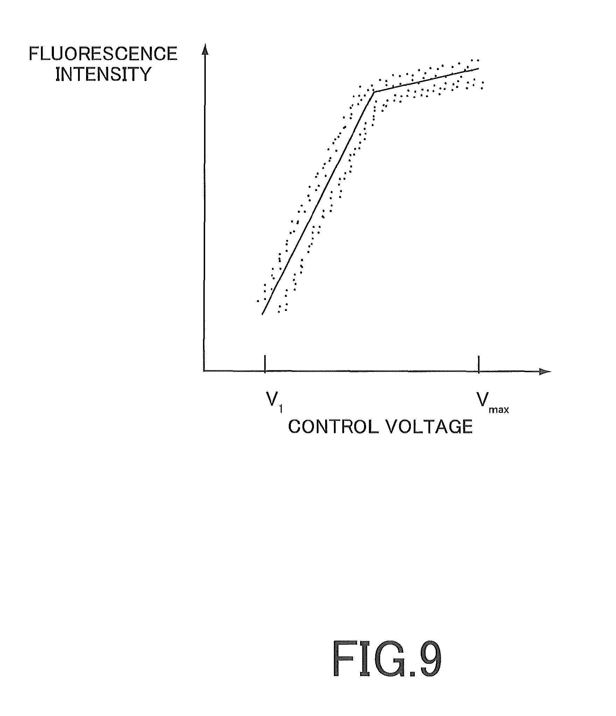

The determination as to whether the fluorescence signal outputted from the PMT 27 is saturated or not may be made according to input given by an operator who has checked a graph displayed on a display (not shown) as a result of characteristics measurement. Alternatively, to determine whether the fluorescence signal is saturated or not, the analyzing device 80 may automatically analyze a graph illustrating a result of characteristics measurement displayed on a display (not shown). For example, as illustrated in Fig. 9, when a graph of the fluorescence intensity calculated by the fluorescence intensity calculating unit 92 versus the control voltage V is displayed as a result of characteristics measurement, the determination as to whether the fluorescence signal is saturated or not is made by determining whether or not there is a region where a linear straight line representing the fluorescence intensity-control voltage V characteristics bends and changes so that its gradient becomes lower with increase in the control voltage based on a result visually observed by an operator or on an analysis result obtained by the analyzing device 80. When the determination result is positive, the calibration process is returned to Step S20, and the steps from Step 20 to Step 80 are repeated.

When the determination result in Step 90 is negative, that is, when the PMT 27 is not in a saturated state, the output level adjusting unit 94 extracts the value of the constant X and the value of the constant A using a regression formula by approximation (Step S100) and the extracted value of the constant X and the extracted value of the constant A are stored in the memory 84 (Step S110). As the regression formula, the following formula (5) obtained by rearranging the above formula (4) is used.

$$\theta_{meas} = X - \theta_\tau - A \cdot V^{(-1/2)} \qquad (5)$$

wherein $\theta_\tau = \tan^{-1}(2\pi f\tau)$, f is the frequency (known) used to modulate the intensity of the laser light, and $\tau$ is the fluorescence relaxation time (known) of the fluorescent protein $X_1$ for use in measurement.

The output level adjusting unit 94 extracts the value of the constant X and the value of the constant A from the measured phase shift angle $\theta_{meas}$ using a regression formula such as the above formula (5) so that the phase shift angles $\theta_{meas}$ predicted using the above formula (5) approximate the measured phase shift angles $\theta_{meas}$. In other words, the output level adjusting unit 94 extracts the value of the constant X and the value of the constant A so that the phase shift angle obtained by correcting the measured phase shift angle $\theta_{measn}$ using the above formula (4) (the right hand-side value of the formula (4)) approximates the phase shift angle $\theta_\tau$ determined using the known fluorescence relaxation time $\tau$. More specifically, the output level adjusting unit 94 extracts the value of the constant X and the value of the constant A so that a fluorescence relaxation time calculated from the corrected phase shift angle expressed on the right hand-side of the formula (4) approximates the known fluorescence relaxation time $\tau$.

In the case illustrated in Fig. 7A, 3.7584 is extracted as the value of the constant X and 0.6360 is extracted as the value of the constant A. The value of the constant X and the value of the constant A are extracted using a well-known method such as a least squares approximation method. The value of the constant X and the value of the constant A are not dependent on the fluorescent protein $X_1$ and specific to the apparatus, and therefore can be used even when another kind of fluorescent protein is used.

As illustrated in Figs. 7A and 7B, the analyzing device 80 can accurately predict the change of the phase shift angle $\theta_{meas}$ with respect to the control voltage of the PMT 27 using the set regression formula. Therefore, the analyzing device 80 can calculate the phase shift angle $\theta_\tau$ derived from the fluorescent protein $X_1$ from the measured phase shift angle $\theta_{meas}$ using the correction formula (4) obtained by setting value of the constant X and value of the constant A included in the regression formula. That is, the analyzing device 80 can correct the phase shift angle $\theta_{meas}$.

Therefore, the analyzing device 80 can accurately calculate the fluorescence relaxation time of fluorescence emitted from the fluorescent protein $X_1$.

The fluorescence detecting apparatus, the fluorescence detecting method, and the fluorescence signal processing method according to the present invention have been described above in detail, but the present invention is not limited to the above embodiment. It is apparent that various changes and modifications may be made without departing from the scope of the present invention.

Reference Signs List

| | |
|---|---|
| 10 | flow cytometer |
| 12 | sample |
| 20 | signal processing device |
| 22 | laser light source unit |
| 22a | light source |
| 23, 26a | lens system |
| 24, 26 | light-receiving unit |
| 26d | voltage adjusting section |
| 27 | photomultiplier tube |

| 27a | cathode |
| 27b | focusing electrode |
| 27c | electron-multiplier section |
| $27c_1$ to $27c_{11}$ | dynode |
| $27d_1$ to $27d_{11}$ | anode |
| 27e | stem |
| 28 | control·processing unit |
| 30 | tube |
| 32 | recovery container |
| 34 | laser driver |
| 40 | signal generating section |
| 42 | signal processing section |
| 44 | signal control section |
| 46 | oscillator |
| 48 | power splitter |
| 50, 52, 54, 64 | amplifier |
| 58 | IQ mixer |
| 60 | system controller |
| 62 | low-pass filter |
| 66 | A/D converter |
| 80 | analyzing device |
| 82 | CPU |
| 84 | memory |
| 86 | phase shift calculating unit |
| 88 | phase shift correcting unit |
| 90 | fluorescence relaxation time calculating unit |
| 92 | fluorescence intensity calculating unit |
| 94 | output level adjusting unit |

**Claims**

1. A fluorescence detecting apparatus for processing a fluorescence signal of fluorescence emitted from an object to be measured irradiated with laser light, the apparatus comprising:

   a light source unit for emitting laser light whose intensity is modulated using a modulation signal with a predetermined frequency;
   a light-receiving unit provided with an output level-adjustable light-receiving element for receiving fluorescence emitted from an object to be measured irradiated with the laser light and for outputting a fluorescence signal at an adjusted output level;
   a first processing unit for mixing the fluorescence signal and the modulation signal with the frequency to generate fluorescence data including information about phase and intensity; and
   a second processing unit for calculating a first phase shift of the fluorescence with respect to the modulation signal using the fluorescence data, calculating a second phase shift by making a correction to the calculated first phase shift depending on conditions for adjusting the output level, and calculating a fluorescence relaxation time of the fluorescence using the calculated second phase shift.

2. The fluorescence detecting apparatus according to claim 1, wherein the light-receiving unit includes an adjusting section for adjusting the output level using an adjustment parameter value given to the light-receiving element.

3. The fluorescence detecting apparatus according to claim 2, wherein the second processing unit is configured to use a correction formula determined depending on the adjustment parameter value for making the correction.

4. The fluorescence detecting apparatus according to claim 3, wherein the second processing unit is configured to previously store a value of a constant used in the correction formula, and to call and use the value of the constant for making the correction.

5. The fluorescence detecting apparatus according to claim 3 or 4, wherein the second processing unit is provided with an output level adjusting section for extracting the value of the constant used in the correction formula when a relationship between the adjustment parameter value and the output level indicates that the output level of the light-receiving element is not saturated, the relationship being determined by changing, in advance, the adjustment parameter value given to the light-receiving element.

6. The fluorescence detecting apparatus according to claim 5, wherein when a reference object having a known fluorescence relaxation time is used as the object to be measured, the output level adjusting section is configured to extract the value of the constant so that the fluorescence relaxation time calculated from the second phase shift approximates the known fluorescence relaxation time of the reference object by the correction.

7. The fluorescence detecting apparatus according to any one of claims 2 to 6, wherein the light-receiving element is a photomultiplier tube and the adjustment parameter value is a voltage applied to an electrode of the photomultiplier tube.

8. The fluorescence detecting apparatus according to claim 7, wherein when the voltage applied to the electrode is represented by V, the first phase shift is represented by $\theta_{meas}$, and the second phase shift is represented by $\theta_\tau$, the correction formula is represented by the following formula: $\theta_\tau = X - \theta_{meas} - A \cdot V^{(-1/2)}$, where X and A are constants.

9. The fluorescence detecting apparatus according to any one of claims 1 to 8, wherein an adjustable neutral density filter is provided in front of a light-receiving surface of the light-receiving element.

10. A fluorescence detecting method for use in a fluorescence detecting apparatus for processing a fluorescence signal of fluorescence emitted from an object to be measured irradiated with laser light, the method comprising the steps of:

emitting laser light whose intensity is modulated using a modulation signal with a predetermined frequency;
receiving by a light-receiving element fluorescence emitted from an object to be measured irradiated with the laser light and outputting a fluorescence signal at an adjusted output level;
mixing the fluorescence signal and the modulation signal with a predetermined frequency to generate fluorescence data including information about phase and intensity; and
calculating a first phase shift of the fluorescence with respect to the modulation signal using the fluorescence data, calculating a second phase shift by making a correction to the calculated first phase shift depending on conditions for adjusting the output level, and calculating a fluorescence relaxation time of the fluorescence using the calculated second phase shift.

11. A method for allowing a fluorescence detecting apparatus to process a fluorescence signal of fluorescence emitted from a reference object having a known fluorescence relaxation time by irradiation with laser light, the method comprising:

a first step of emitting laser light whose intensity is modulated using a modulation signal with a predetermined frequency;
a second step of receiving by a light-receiving element fluorescence emitted from a reference object irradiated with the laser light and outputting a fluorescence signal at an adjusted output level;
a third step of mixing the fluorescence signal and the modulation signal with a predetermined frequency to generate fluorescence data including information about phase and intensity;
a fourth step of calculating a first phase shift of the fluorescence with respect to the modulation signal using the fluorescence data and storing a pair of the calculated first phase shift and an adjustment parameter value given to the light-receiving element to adjust the output level;
a fifth step of storing two or more pairs of the first phase shift and the adjustment parameter value by repeating the first to fourth steps while varying the adjustment parameter value;
a sixth step of calling the two or more stored pairs and approximating a regression formula expressing a relationship between the adjustment parameter value and the first phase shift to a relationship represented by the two or more called pairs so as to extract a value of an unknown constant included in the regression formula; and
a seventh step of storing the extracted value of the constant.

12. The fluorescence signal processing method according to claim 11, wherein the adjustment parameter value is changed so that the output level is reduced every time the first to fourth steps are repeated.

13. The fluorescence signal processing method according to claim 11 or 12, wherein the fluorescence to be received by the light-receiving element is attenuated by a neutral density filter before being received by the light-receiving element so that the output level of the fluorescence signal outputted by the light-receiving element is not saturated.

## Amended claims under Art. 19.1 PCT

1. A fluorescence detecting apparatus for processing a fluorescence signal of fluorescence emitted from an object to be measured irradiated with laser light, the apparatus comprising:

a light source unit for emitting laser light whose intensity is modulated using a modulation signal with a predetermined frequency;
a light-receiving unit provided with an output level-adjustable light-receiving element for receiving fluorescence emitted from an object to be measured irradiated with the laser light and for outputting a fluorescence signal at an adjusted output level;
a first processing unit for mixing the fluorescence signal and the modulation signal with the frequency to generate fluorescence data including information about phase and intensity; and
a second processing unit for calculating a first phase shift of the fluorescence with respect to the modulation signal using the fluorescence data, calculating a second phase shift by making a correction to the calculated first phase shift depending on conditions for adjusting the output level, and calculating a fluorescence relaxation time of the fluorescence using the calculated second phase shift.

2. The fluorescence detecting apparatus according to claim 1, wherein the light-receiving unit includes an adjusting section for adjusting the output level using an adjustment parameter value given to the light-receiving element.

**3.** The fluorescence detecting apparatus according to claim 2, wherein the second processing unit is configured to use a correction formula determined depending on the adjustment parameter value for making the correction.

**4.** The fluorescence detecting apparatus according to claim 3, wherein the second processing unit is configured to previously store a value of a constant used in the correction formula, and to call and use the value of the constant for making the correction.

**5.** The fluorescence detecting apparatus according to claim 3 or 4, wherein the second processing unit is provided with an output level adjusting section for extracting the value of the constant used in the correction formula when a relationship between the adjustment parameter value and the output level indicates that the output level of the light-receiving element is not saturated, the relationship being determined by changing, in advance, the adjustment parameter value given to the light-receiving element.

**6.** The fluorescence detecting apparatus according to claim 5, wherein when a reference object having a known fluorescence relaxation time is used as the object to be measured, the output level adjusting section is configured to extract the value of the constant so that the fluorescence relaxation time calculated from the second phase shift approximates the known fluorescence relaxation time of the reference object by the correction.

**7.** The fluorescence detecting apparatus according to any one of claims 2 to 6, wherein the light-receiving element is a photomultiplier tube and the adjustment parameter value is a voltage applied to an electrode of the photomultiplier tube.

**8.** The fluorescence detecting apparatus according to claim 7, wherein when the voltage applied to the electrode is represented by V, the first phase shift is represented by $\theta_{meas}$, and the second phase shift is represented by $\theta_\tau$, the correction formula is represented by the following formula: $\theta_\tau = X - \theta_{meas} - A \cdot V^{(-1/2)}$, where X and A are constants.

**9.** The fluorescence detecting apparatus according to any one of claims 1 to 8, wherein an adjustable neutral density filter is provided in front of a light-receiving surface of the light-receiving element.

**10.** A fluorescence detecting method for use in a fluorescence detecting apparatus for processing a fluorescence signal of fluorescence emitted from an object to be measured irradiated with laser light, the method comprising the steps of:

emitting laser light whose intensity is modulated using a modulation signal with a predetermined frequency;
receiving by a light-receiving element fluorescence emitted from an object to be measured irradiated with the laser light and outputting a fluorescence signal at an adjusted output level;
mixing the fluorescence signal and the modulation signal with a predetermined frequency to generate fluorescence data including information about phase and intensity; and
calculating a first phase shift of the fluorescence with respect to the modulation signal using the fluorescence data, calculating a second phase shift by making a correction to the calculated first phase shift depending on conditions for adjusting the output level, and calculating a fluorescence relaxation time of the fluorescence using the calculated second phase shift.

**11.** (Cancelled)

**12.** (Cancelled)

**13.** (Cancelled)

**14.** (Added) The fluorescence detecting method according to claim 10,
wherein the output level of the outputted fluorescence signal is adjusted by an adjustment parameter value given to the light-receiving element,
wherein when the second phase shift is calculated, the correction is made depending on conditions for adjusting the output level by using a correction formula which is determined depending on the adjustment parameter value and to which a value of a previously-stored constant is given,
wherein the value of the constant given to the correction formula is extracted so that a fluorescence relaxation time calculated from the second phase shift by using a reference object having a known fluorescence relaxation time as the object to be measured approximates the known fluorescence relaxation time of the reference object by the correction, and
wherein the extraction of the value of the constant is performed when a relationship between the adjustment parameter value and the output level indicates that the output level of the light-receiving element is not saturated, the relationship being determined by changing, in advance, the adjustment parameter value given to the light-receiving element.

**15.** (Added) The fluorescence detecting method according to claim 10 or 14, wherein an adjustable neutral density filter is provided in front of a light-receiving surface of the light-receiving element, and the neutral density filter is adjusted so that the output level of the light-receiving element is not saturated.

FIG.1

LIGHT SOURCE

L

22a

34:DRIVER

60

23

30

12

22

# FIG.2

30

12

26

FL

26b

26a

26c

26d

27

54

VOLTAGE ADJUSTING SECTION

94

94

# FIG.3

FIG.4

28

40:SIGNAL
GENERATING
SECTION

50

34 ◄── AMP ◄── POWER
SPLITTER ◄── f ── OSCILLATOR

48

46

AMP ~52

34

SYSTEM
CONTROLLER

60

27 ── AMP ── IQ MIXER

54

58

LOW-PASS
FILTER

62

64

AMP

66

A/D ── 86

42:SIGNAL
PROCEESSING
SECTION

44:SIGNAL
CONTROL
SECTION

# FIG.5

80 : ANALYZING DEVICE

| FLUORESCENCE INTENSITY CALCULATING UNIT | ~92 |

66

| PHASE SHIFT CALCULATING UNIT | ~86 |

$\theta_{meas}$

| PHASE SHIFT CORRECTING UNIT |

| FLUORESCENCE RELAXATION TIME CALCULATING UNIT | 90 |

88

OUTPUT DEVICE

26b
26b
26d

| OUTPUT LEVEL ADJUSTING UNIT | ~94 |

| CPU | ~82 |

| MEMORY | ~84 |

# FIG.6

PHASE SHIFT
ANGLE $\theta_{meas}$
(rad)

FIG.7A

CONTROL VOLTAGE (V)

PHASE SHIFT
ANGLE $\theta_{meas}$
(rad)

FIG.7B

CONTROL VOLTAGE (V)

FIG.8

FIG.9

## INTERNATIONAL SEARCH REPORT

| | International application No. |
| --- | --- |
| | PCT/JP2011/000004 |

**A. CLASSIFICATION OF SUBJECT MATTER**
*G01N21/64*(2006.01)i, *G01N15/14*(2006.01)i, *G01N33/483*(2006.01)i

According to International Patent Classification (IPC) or to both national classification and IPC

**B. FIELDS SEARCHED**

Minimum documentation searched (classification system followed by classification symbols)
G01N21/00-21/74, G0J1/00-1/60, G01J11/00

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| Y<br>A | Shigeyuki TANAKA et al., "Development of fluorescence lifetime FRET flow cytometer", Mitsui Zosen Technical Review, 31 March 2007 (31.03.2007), no.190, pages 54 to 60 | 1-5,7-10<br>6,11-13 |
| Y<br>A | Hamamatsu Photonics Kabushiki Kaisha Henshu Iinkai, Hikari Denshi Zobaikan sono Kiso to Oyo, 3a-th edition, Hamamatsu Photonics Kabushiki Kaisha, 01 July 2007 (01.07.2007), pages 48 to 53 | 1-5,7-10<br>6.11-13 |
| A | JP 62-091826 A (Hamamatsu Photonics Kabushiki Kaisha), 27 April 1987 (27.04.1987), entire text; all drawings & US 4709140 A | 1-13 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 March, 2011 (24.03.11) | 05 April, 2011 (05.04.11) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

| | International application No. |
|---|---|
| | PCT/JP2011/000004 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2008-256380 A (Hamamatsu Photonics Kabushiki Kaisha), 23 October 2008 (23.10.2008), entire text; all drawings (Family: none) | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006226698 A